# EUROPEAN PATENT APPLICATION

(11) **EP 2 602 733 A2**
(43) Date of publication of application: **12.06.2013**
(21) Application number: 11193637.3
(22) Date of filing: 15.12.2011
(51) Int. Cl.: G06F 19/18

(54) **Biological cell assessment using whole genome sequence and oncological therapy planning using same**

(30) Priority: 08.12.2011 US 201161568262 P
(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

A cancer test includes: processing a suspect tissue sample **(10)** acquired from a subject **(6)** to generate a suspect whole genome sequence (WGS) **(20)**; processing a normal tissue sample **(12)** acquired from the subject to generate a normal WGS **(22)**; computing a WGS comparison metric comparing the suspect WGS with the normal WGS; and identifying whether the suspect tissue sample comprises cancer tissue based on the computed WGS comparison metric. A tumor delineation method comprises: acquiring a plurality of probative tissue samples **(104)** from a subject **(6)** in or near a tumor **(100)**; recording the sampling locations of the probative tissue samples; classifying each probative tissue sample respective to cancer based on genetic testing of the probative tissue sample; and delineating a boundary **(110)** of the tumor based on the classifications of the probative tissue samples and the recorded sampling locations.

## Description

The following relates to the medical arts, oncology arts, genomic arts, and related arts. It is described with particular reference to oncological tumor delineation applications; however, the following is more generally applicable in medical or veterinary research and development, screening, diagnosis, clinical monitoring of metastasis or other conditions, interventional planning, and other medical or veterinary applications directed toward oncological conditions and other adverse conditions.

Cancer arises when normal body cells mutate or otherwise transform into cancerous cells that divide and multiply in an uncontrolled manner. In some cancers the cancerous cells remain localized, at least initially, so as to form a malignant tumor which often invades surrounding tissue with micro infiltrations. At this point the cancer can sometimes be treated by removing the tumor; however, such removal should be complete otherwise the remaining cancer cells can continue to multiply and lead to a recurrence of the cancer. In addition to surgical removal, an adjuvant and/or neoadjuvant therapy or therapies may be applied, such as radiation therapy, chemotherapy, or so forth, which may address any incompleteness of the malignant tissue removal. A cancer metastasizes when it becomes delocalized and spreads to substantial portions of the body through the bloodstream or through the lymphatic system. Metastatic cancer is typically treated by administration of drugs (chemotherapy) or radiation in the form of radioactive implants (brachytherapy) or direct application of ionizing radiation (radiation therapy). These techniques may also be used prior to metastasis, either instead of surgical tumor removal in cases for which surgical removal of the malignancy is contraindicated, or in addition to surgical tumor removal to cull any cancer cells that remain after the tumor removal.

A known tool for cancer identification is genetic analysis. Typically, this entails performing genotyping to identify whether a suspect cell includes a particular genetic variant, or combination of variants, that has (have) been shown in clinical studies to correlate with a type of cancer. Ongoing oncology research is continually expanding the database of such genetic signatures for identifying various types of cancer.

The effectiveness of these genetic approaches is contingent upon there being a known genetic signature for the specific cancer condition of the subject (e.g., human oncology patient or veterinary oncology subject) under investigation. This may not always be the case. Some variants that are actually related to cancer may be novel (e.g., specific to a particular subject and not generally observed in the pool of patients with that cancer), or may be population specific (e.g., specific to a particular ethnic group, gender, geographical region, or so forth).

Although the number of variant-cancer correlations identified in the oncology literature is always expanding, which should in principle, increase the effectiveness of genetic analysis for cancer diagnosis, there are practical limitations. The adoption of newly published variants for clinical diagnosis and monitoring can be delayed by concerns about validation and/or by government regulatory delays. Moreover, a larger variant database translates into longer processing time as more and more variants must be acquired and tested. Acquisition delays can be reduced by acquiring a whole genome sequence (WGS) using advanced sequencing technologies. The downstream processing delays, however, are not reduced by WGS acquisition.

Moreover, the variants database cannot encompass unique (or nearly unique) variants that occur in a portion of the cancer pool that is too small to be statistically detectable in clinical studies. A larger variants database also increases the likelihood of ambiguous or irreconcilable data, such as studies drawing contradictory conclusions as to the correlation (or lack thereof) between a particular variant and a particular cancer. In such cases existing genetic analyses are unlikely to yield a clinically useful result.

The following contemplates improved apparatuses and methods that overcome the aforementioned limitations and others.

According to one aspect, a method comprises: processing a suspect tissue sample acquired from a subject to generate a suspect whole genome sequence; processing a normal tissue sample acquired from the subject to generate a normal whole genome sequence; computing a whole genome sequence comparison metric comparing the suspect whole genome sequence with the normal whole genome sequence; and identifying whether the suspect tissue sample comprises cancer tissue based on the computed whole genome sequence comparison metric.

According to another aspect, a non-transitory storage medium stores instructions executable by an electronic data processing device to perform a method as set forth in the immediately preceding paragraph. According to another aspect, an apparatus comprises an electronic data processing device configured to perform a method as set forth in the immediately preceding paragraph. According to another aspect, a method as set forth in the immediately preceding paragraph further comprises: acquiring tissue samples from the subject at a plurality of sampling locations in or near a tumor; recording the sampling locations; performing the processing, computing, and identifying for each tissue sample; and delineating a boundary of the tumor based on the identifying and the recorded sampling locations.

According to another aspect, a method comprises: classifying tissue samples acquired from a subject at sampling locations in or near a tumor respective to cancer based on genetic testing of the tissue samples; and delineating a boundary of the tumor based on the classifying and knowledge of the sampling locations from which the samples were acquired.

According to another aspect, a method comprises: acquiring a plurality of probative tissue samples from a subject in or near a tumor; recording the sampling locations of the probative tissue samples; classifying each probative tissue sample respective to cancer based on genetic testing of the probative tissue sample; and delineating a boundary of the tumor based on the classifications of the probative tissue samples and the recorded sampling locations.

One advantage resides in providing identification of cancer cells based on WGS data with sufficient rapidity for use in time-critical clinical application such as tumor delineation preparatory to an interventional oncology procedure.

Another advantage resides in providing cancer cell identification based on WGS that is not reliant upon calling specific cancer-correlative variants.

Another advantage resides in providing broad-based cancer cell identification that is not limited to specific known cancer types having identified correlative genetic variants.

Another advantage resides in providing tumor delineation that is not dependent upon the cancer cells exhibiting distinctive morphology or staining characteristics.

Numerous additional advantages and benefits will become apparent to those of ordinary skill in the art upon reading the following detailed description.

The invention may take form in various components and arrangements of components, and in various process operations and arrangements of process operations. The drawings are only for the purpose of illustrating preferred embodiments and are not to be construed as limiting the invention.
FIGURE 1 diagrammatically shows a sample extraction laboratory and a genomics laboratory suitably configured to perform cancer cell identification based on whole genome sequence (WGS) information as disclosed herein.
FIGURES 2-5 diagrammatically show various embodiments of the WGS comparison metric calculation and cancer cell identification methodology using same.
FIGURE 6 diagrammatically shows acquisition of probative tissue samples from a subj ect at sampling locations in or near a tumor for use in interventional procedure planning as disclosed herein.

Existing genetic analyses correlate observable genetic variants with specific types of cancer. This approach assumes that cancers fall into well-defined types, and that a given type of cancer can be characterized by correlative genetic variants that are common to patients (or veterinary subj ects, in the veterinary context) having that type of cancer.

However, it is recognized herein that these assumptions may not be met in many situations. For example, reported studies in both oestrogen receptor-positive and oestrogen receptor-negative breast cancer have shown that substantial complexity and heterogeneity is actually observed between cancer genomes from different patients with the same breast cancer histopathological phenotype (inter-tumoural heterogeneity). See Shah et al., "Mutational evolution in a lobular breast tumour profiled at single nucleotide resolution", Nature vol. 461 pages 809-813 (2009); Stephens et al., "Complex landscapes of somatic rearrangement in human breast cancer genomes", Nature vol. 462 pages 1005-1010 (2009); and Ding et al., "Genome remodelling in a basal-like breast cancer metastasis and xenograft", Nature vol. 464, pages 999-1005 (2010). For example, none of the novel fusion genes identified by Stephens et al. were present more than once in any of the twenty-four cancers studied, and three expressed in-frame fusion genes selected for follow-up were not present in an additional 288 breast cancers studied as reported in Shah et al. Another study has described substantial heterogeneity within individual breast tumors (intra-tumoral heterogeneity), where multiple tumor subpopulations have been identified, each with distinct genomic profiles. *See* Navin et al., "Inferring tumor progression from genomic heterogeneity", Genome Res. Vol. 20 pages 68-80 (2010).

Moreover, it is known that differences in variant-cancer correlation can occur between populations, such that genomic signatures (e.g., mutations, single-nucleotide polymorphisms i.e. SNPs, insertions or deletions i.e. indels, etc.) reported in literature for a particular population may be inappropriate for use in the other population. For example, in one study of sequence variants flagged as disease mutations, 74% of the studied variants turned out to be polymorphisms. Still further, even if a mutation is cited in literature as correlating with a certain type of cancer, this does not guarantee that it indeed is the causative mutation. In fact 27% of the cited disease mutations were found to be likely polymorphisms or to be misannotated in the same study.

Indeed, the conventional model for carcinogenesis, namely a gradual accumulation of individual, relatively discrete genetic mutations transitioning normal cells into cancer cells, has been challenged. For example, a recently developed model for some instances of carcinogenesis is chromothripsis. In this model, a chromosome undergoes large scale fracturing followed by inaccurate reassembly. Stephens et al., "Massive Genomic Rearrangement Acquired in a Single Catastrophic Event during Cancer Development", Cell viol. 14 no. 1 pages 27-40 (January 2011). The chromothripsis model does not predict that a particular type of cancer would be likely to be associated with correlative discrete genetic variants. Another model that is becoming popular hypothesizes driver and passenger mutations. This model is based on the observation that many cancer genomes are riddled with mutations. In this model, the vast majority of these mutations are likely to be passengers - that is, mutations that do not contribute to the development of cancer but instead have occurred during the growth of the cancer. See http://www.news-medical.net/news/20100219/Cancer-genomes-Distinguishing-between-driver-and-passenger-mutations.aspx (last accessed October 27, 2011). According to this model, most of the mutations in the biological databases will be passenger mutations.

Cancer identification techniques disclosed herein reduce or eliminate reliance upon literature-based cancer-correlative genetic variants. The disclosed techniques rely instead upon first principles considerations that are expected to be valid for all cancers regardless of the carcinogenesis mechanism. The disclosed techniques also leverage the availability of a whole genome sequence (WGS) which is provided by some existing commercially available genome sequencers or sequencing services (suitable sequencers or sequencing services are available, for example, from: Illumina^{Ⓡ}, San Diego, CA, USA; Knome^{Ⓡ}, Cambridge, MA, USA; Roche 454 (available from Roche, Basel, Switzerland); and Ion Torrent, Guilford, Connecticut, USA.

The techniques disclosed herein are premised on the following observation: All cancers are associated with abnormal changes to the genome. This is true regardless of the particular mechanism of carcinogenesis, and regardless of the particular type of cancer. Based on this observation, the disclosed techniques rely upon comparison of the WGS of a suspect cell with the WGS of a normal cell from the same individual. If the suspect cell is indeed a cancer cell, then the difference between its WGS and the WGS of a normal cell from the same individual is expected to be larger than the difference between the WGS of two different normal cells from the same individual. Thus, by comparing the WGS of a suspect tissue sample taken from a subject (e.g., a human medical subject, or a veterinary subject) with the WGS of a normal tissue sample taken from the same subject, the likelihood that the suspect tissue sample actually comprises cancer tissue is readily assessed. The WGS of normal tissue is employed as a filter to remove portions of the genome that are unrelated to cancer, leaving only the unique variants that are probative of whether the suspect tissue is actually cancer tissue.

This approach has substantial advantages. It substantially reduces the likelihood of misinterpreting a benign (i.e., not cancer-related) variant as a cancer signature, since such benign variants will be filtered out by comparison with the normal WGS of the same subject. On the other hand, a unique cancer-related variant that would not be detected by comparison with variant-cancer correlates from the literature is readily detected using the disclosed approach.

The disclosed approach determines whether the suspect tissue sample comprises cancer; however, it does not identify which type of cancer. The skilled artisan might view this as a substantial disadvantage for cancer diagnosis and monitoring. However, it is recognized herein that this potentially perceived disadvantage is not as substantial as might initially be thought. First, because the disclosed approaches do not rely upon exhaustive comparison of genetic material with a reference database of variants, they are substantially faster than conventional variant-based cancer identification. Thus, they can be used in initial cancer screening (with follow-up in the form of a conventional variant-based cancer identification in cases where the disclosed approach indicates a likelihood of cancer). The disclosed approaches are also useful in cancer monitoring, since in that case the type of cancer is (usually) already known and the information being sought is the progression of the cancer. As further disclosed herein, the speed of the disclosed approaches for even make them viable techniques for use in delineating a tumor during planning for an interventional procedure such as surgical removal or radiation therapy.

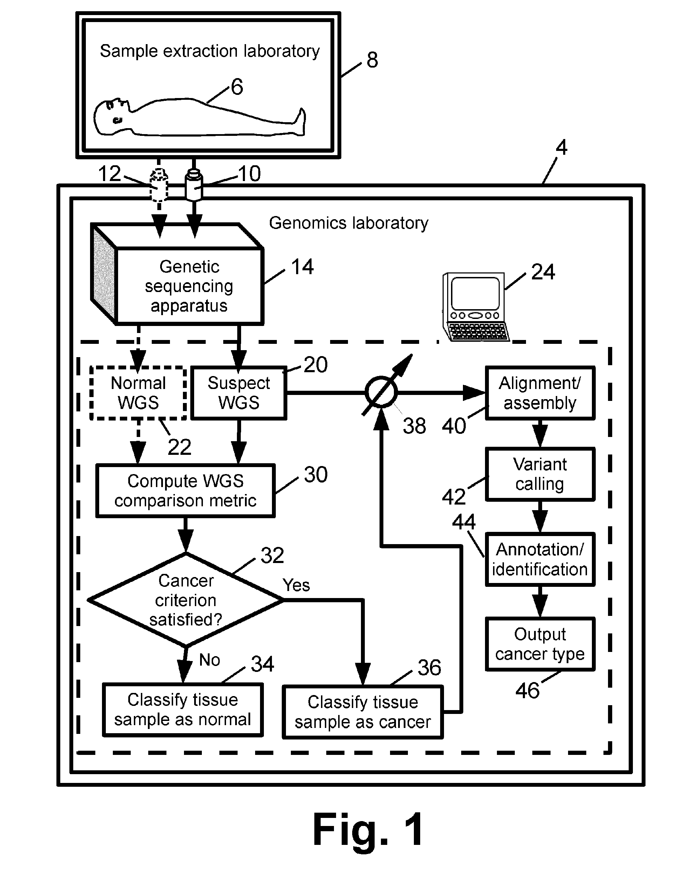

With reference to FIGURE 1, the disclosed cancer testing techniques are suitably performed by a genomics laboratory **4** performing the disclosed cancer testing on one or more tissue samples extracted from a patient **6** in a sample extraction laboratory **8**. It is to be appreciated that the laboratories **4**, **8** may have various relationships. For example, in some embodiments the two laboratories **4**, **8** are the same laboratory, e.g. an in-house genomics laboratory at a hospital that also performs its own tissue sampling. In other embodiments, the two laboratories **4**, **8** may be different in-house laboratories located at the same hospital or other common medical facility. In yet other embodiments the two laboratories **4**, **8** may be different organizationally and/or geographically. For example, the sampling laboratory **8** may be an in-house laboratory located at a hospital, while the genomics laboratory **4** may be a commercial service provider that receives the extracted tissue sample via mail or other delivery pathway and communicates the test results back to the hospital via the Internet or another electronic communication pathway.

In any of these embodiments, the sampling laboratory **8** extracts at least two tissue samples from the subject **6**, namely a "suspect" tissue sample **10** and a "normal" tissue sample **12**. The suspect tissue sample **10** is a tissue sample acquired from a location or region of the subject **6** that is suspected of comprising cancer tissue. For example, the suspect tissue sample **10** may be acquired from a tumor suspected or known to be malignant (it is to be understood that as used herein "suspected" encompasses "known"), or from a lung suspected to have lung cancer, or from a breast cancer lesion known or suspected to be malignant, or so forth. The normal tissue sample **12** is acquired from the same subject **6**, but from a region or location of the subject **6** that is effective to ensure that the normal tissue sample **12** does not comprise cancer tissue. The identification of such a "normal" region from which the normal tissue sample **12** may be extracted can be based on various types of information. For example, in the case of a malignant tumor that has not (yet) metastasized the normal tissue sample **12** can be safely drawn from a location of the same type of tissue that is sufficiently far away from the tumor that it is unlikely to contain a non-negligible quantity of cancer cells. In the case of metastatic cancer, the normal tissue sample **12** may be drawn from tissue of a type that is unlikely to contain a non-negligible quantity of metastasized cancer cells. For example, if the cancer is unlikely to have spread to oral tissue, then the normal tissue sample **12** may be an oral sample. In general, the suspect tissue sample **10** and the normal tissue sample **12** may or may not be of the same tissue type.

It will be noted that in illustrative FIGURE 1 the samples **10**, **12** are represented by vials; however, it is to be understood that the samples **10**, **12** may in general take any form suitable for the type of tissue that has been sampled, and may be contained or supported by any suitable container or support for that type of tissue. For example, the samples **10**, **12** may be fluid samples (e.g., blood) acquired using a hypodermic needle or other fluid collection apparatus, surface samples (e.g. obtained by oral swabs and disposed on a sterile slide or other suitable surface), biopsy samples acquired using a biopsy needle or other interventional instrument, or so forth. (As an aside, in the drawings, for visual enhancement the normal tissue sample **12** and processing that utilizes only the normal tissue sample **12** are drawn using dashed lines.) Still further, while the illustrative suspect tissue sample **10** is represented as a single sample and the illustrative normal tissue sample **12** is represented as a single sample, it is to be understood that either or both samples may actually comprise a set of two or more samples whose results are averaged or otherwise combined.

The tissue samples **10**, **12** are conveyed from the sampling laboratory **8** to the genomics laboratory **4** (unless the laboratories **4**, **8** are the same physical establishment). At the genomics laboratory **4**, each sample **10**, **12** is suitably prepared and processed using a genetic sequencing apparatus **14** to generate a suspect whole genome sequence (suspect WGS) **20** and a normal whole genome sequence (normal WGS) **22**, corresponding to the suspect tissue sample **10** and the normal tissue sample **12** respectively. The genetic sequencing apparatus **14** can employ substantially any sequencer that is capable of generating a whole genome sequence (WGS). Some suitable sequencing apparatus are available from Illumina^{Ⓡ}, San Diego, CA, USA; Knome^{Ⓡ}, Cambridge, MA, USA; Roche 454 (available from Roche, Basel, Switzerland); and Ion Torrent, Guilford, Connecticut, USA.

As used herein, a "whole genome sequence", or WGS (also referred to in the art as a "full", "complete", or entire" genome sequence), or similar phraseology is to be understood as encompassing a substantial, but not necessarily complete, genome of a subject. In the art the term "whole genome sequence", or WGS is used to refer to a nearly complete genome of the subject, such as at least 95% complete in some usages. The term "whole genome sequence", or WGS as used herein does not encompass "sequences" employed for gene-specific techniques such as single nucleotide polymorphism (SNP) genotyping, for which typically less than 0.1% of the genome is covered. The term "whole genome sequence", or WGS as used herein does not require that the genome be aligned with any reference sequence, and does not require that variants or other features be annotated.

The WGS **10**, **12** are processed by an electronic data processing device **24**, which in illustrative FIGURE 1 is shown as a representative computer **24**. More generally, the electronic data processing device **24** may be a desktop computer, notebook computer, electronic tablet, network server, or so forth. Moreover, while the illustrative computer **24** is shown as residing inside the genomics laboratory **4**, it is also contemplated for the electronic data processing device to be located outside of the genomics laboratory **4** and to communicate with the laboratory **4** via a wired or wireless local area network, and/or via the Internet, or so forth. For example, the electronic data processing device **24** may be a network server that the laboratory **4** accesses via an electronic hospital network. The processing of the WGS **10**, **12** performed by the electronic data processing device **24** is sometimes referred to as *in silico* processing. It is to be appreciated that various embodiments disclosed herein may be physically embodied as the electronic data processing device **24** programmed or otherwise configured to perform the disclosed *in silico* processing. Further, various embodiments disclosed herein may be physically embodied as a non-transitory storage medium (not shown) storing instructions executable by the electronic data processing device **24** to perform the disclosed *in silico* processing. Such a non-transitory storage medium may, for example, comprise a hard disk or other magnetic storage medium, or an optical disk or other optical storage medium, or a flash memory, random access memory (RAM), read-only memory (ROM), or other electronic storage medium, or so forth.

The disclosed cancer identification tests are based on comparison of the suspect whole genome sequence **20** with the normal whole genome sequence **22**, with the general premise being that the larger the difference is between these WGS **20**, **22** the more likely that the suspect WGS **20** is cancer tissue. In case of cancerous cells, the changes in the genome become more pronounced with large indels (insertions/deletions), wide copy number variations (CNV's), chromosomal aberrations and rearrangements and aneuploidy in extreme cases of highly malignant and dedifferentiated tumor. Again, this is true regardless of the mechanism of carcinogenesis. These genomic changes induce significant alterations or errors in the whole genome, causing the WGS of cancer cells to deviate substantially from the WGS of normal cells. In general, this is a matter of degree. Even the WGS of normal cells is expected to have deviations from one another. These deviations are expected to be substantially larger for cancer cells. This premise can also be applied to monitoring cancer progression from one cancer stage to the next, as the later cancer stages are expected to exhibit more differentiation (versus earlier stage cancer cells) respective to the normal cell WGS. Indeed, WGS of later stage cancer cells are expected to exhibit quantifiable increase in differentiation as compared with the WGS of earlier-stage cancer cells. Advantageously, these changes can be determined even before subjecting the WGS of the suspect tissue sample to the detailed analysis pipeline (e.g., including full alignment/assembly, variant calling and annotation, and comparison with literature variant-cancer correlation databases.

Toward this end, an operation **30** computes a WGS comparison metric providing a quantitative comparison between the suspect whole genome sequence **20** and the normal whole genome sequence **22**. A decision operation **32** determines whether the quantitative WGS comparison metric satisfies a cancer criterion. Depending upon the decision reached at the decision operation **32**, the suspect tissue sample **10** is either classified as normal tissue (operation **34**) or is classified as cancer tissue (operation **36**). In this regard, the decision operation **32** can also be viewed as a classifier or classification operation.

Note that although a binary (i.e., either cancer or normal) classification is employed in the illustrative classifier **32** of FIGURE 1, more generally the classification can employ soft or probabilistic classification (e.g., there is a 70% likelihood that the sample **10** is cancer). In this case, the percentage may be variously interpreted as the probability that the sample **10** contains cancer, or as the "amount" of cancer contained in the sample. For example, the suspect sample **10** may, in actuality, contain some cancer cells and some normal cells. In such a case, a low probability output by the classifier **32** may indicate a low fraction of the cells being cancer cells.

The classifier **32** does not opine as to the type of cancer, but only as to whether or not the suspect sample **10** comprises cancer. The output **34**, **36** may be interpreted and/or utilized in various ways. In the illustrative example of FIGURE 1, the cancer test embodied by the operations **30**, **32**, **34**, **36** is used as a cancer screening test. In this application, if the output **34** is obtained, indicating that the suspect tissue sample **10** is normal tissue, then no further action is typically taken. On the other hand, if the output **36** is obtained, indicating a likelihood of cancer, then additional diagnostics are typically performed under the guidance of a physician.

In the illustrative example of FIGURE 1, the additional diagnostics include performing a conventional genetic variant-cancer correlation analysis. Advantageously, this analysis can "re-use" the suspect WGS **20**. Toward this end, the output **36** serves as an invocation operation **38** that invokes the operations of genome alignment/assembly **40**, variant calling **42** and annotation/identification **44**, and output of cancer type **46** based on the operations **40**, **42**, **44** identifying a genetic variant that has been shown in a clinical study to correlate with that type of cancer. In this embodiment, the additional genetic test **40**, **42**, **44**, **46** serves as both a validation of the cancer test **30**, **32**, **34**, **36** and also provides additional information by identifying the type of cancer.

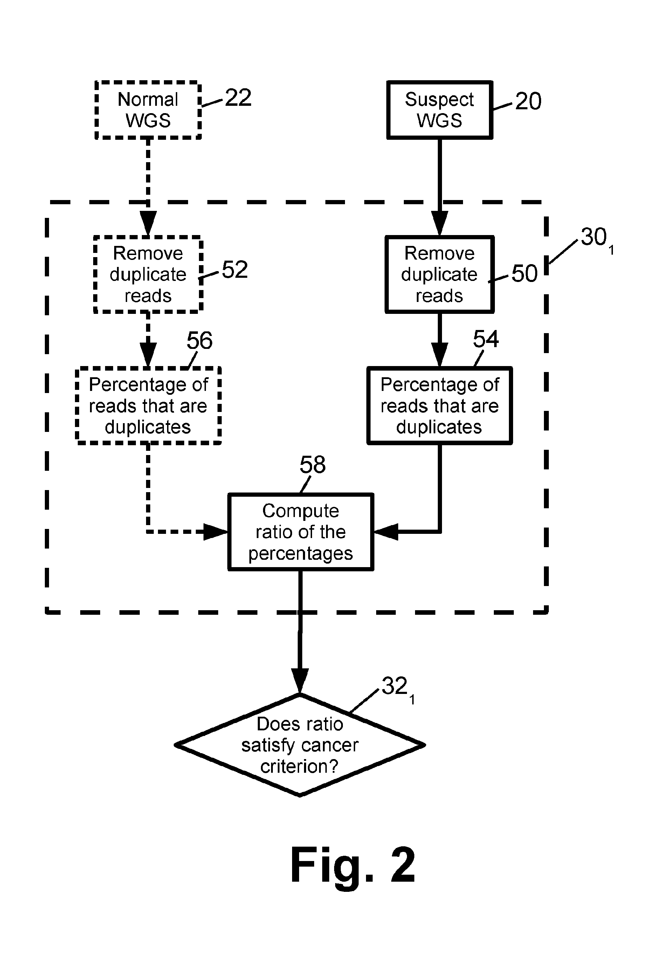

Having provided an overview of the cancer testing techniques disclosed herein with reference to FIGURE 1, some specific embodiments of the WGS comparison metric computation operation **30** and the classifier operation **32** are described with reference to FIGURES 2-5.

With reference to FIGURE 2, a first embodiment **30₁** of the WGS comparison metric computation operation **30** and a first embodiment **32₁** of the classifier operation **32** are described. The suspect WGS **20** is created by sequencing all samples (if more than one) separately to the same coverage and same threshold for base quality applied to select reads for tissue samples in equivalent numbers. The reads per tissue sample is stored in a probabilistic data structure like the Bloom filters. In an operation **50** duplicate reads are removed from the suspect WGS **20**, and in an analogous operation **52** duplicate reads are removed from the normal WGS **22**. It is expected that the reads from the normal cells are not duplicated as much as the reads from cancerous cells, reflecting a higher number of insertions expected for cancer cells as compared with normal cells. Accordingly, in the duplicate read removal operations **50**, **52**, the quantity of removed duplicate reads is quantified by a suitable metric, such as a percentage **54** of reads that are duplicates in the case of the suspect WGS **20** and a percentage **56** of reads that are duplicates in the case of the normal WGS **22**. Based on the percentages **56** for the normal samples (assuming here that there are multiple normal tissue samples that have each been independently sequenced) a threshold is found for the normal cells. In some embodiments a threshold of 10-15% duplicated reads is expected for the normal cells, although a higher or lower value is contemplated based on the measured duplication value **56**. At an operation **58**, a ratio of the percentages **54**, **56** is computed. Any cut-off above (say, more than 20%, corresponding to the carcinogenesis principally comprising duplication inserts) or below (say, less than 10%, corresponding to the carcinogenesis principally comprising deletions) the "normal" percentage **56** may be associated with cancer. The classifier **32₁** then determines whether the ratio computed in operation **58** satisfies the defined cancer criterion, which here is delineated by the aforementioned cut-off values.

The WGS comparison metric computation operation **30₁** described with reference to FIGURE 2 can serve as a fast *in silico* screening test for cancer that does not require alignment of the genome beforehand. One way to efficiently implement the duplicate read detection is through the use of Bloom filters. A Bloom filter comprises an array of bits that are initialized to 0, and a set of hash functions mapping a sequencing read to one of the bits of the array. To add a read to the Bloom filter, the read is hashed by all the hash functions and the output bits are set. To check if a given read has already been added to the Bloom filter (that is, to perform a query), the same process is used except that each output bit is checked to see if it is 1 or 0 - if any checked bit is set to 0 then it is known that the read has not (yet) been added to the Bloom filter, and the check is suitably followed by an add operation to add the read to the filter. *See* "Bloom Filter", http://en.wikipedia.org/wikiBloom filter (last accessed Sept. 23, 2011).

A property of the Bloom filter is that it never erroneously indicates that a read is not in the Bloom filter when it actually is; however, there is a possibility that the Bloom filter may indicate a read is in the filter when it is not. *Id.* This can occur if other add operations have set all of the bits that would have been set by adding the read of the query so that the query returns all 1's even though the read of the query has not actually been added to the Bloom filter. Such an error is not particularly significant for this application, however, because it will only result in the number of duplicate reads being overestimated by one (since the first time the read is checked it will show up as being a duplicate when it is not; thereafter, any repeat of that read check will actually be a duplicate and will be correctly recognized as such). Moreover, the Bloom filter can be fine tuned for the accuracy required and time taken to report by adjusting the number of bits in the array and the number of hash functions.

The WGS comparison metric **30₁** of FIGURE 2 is fast to compute, but does not use much information from the WGS **20**, **22**.

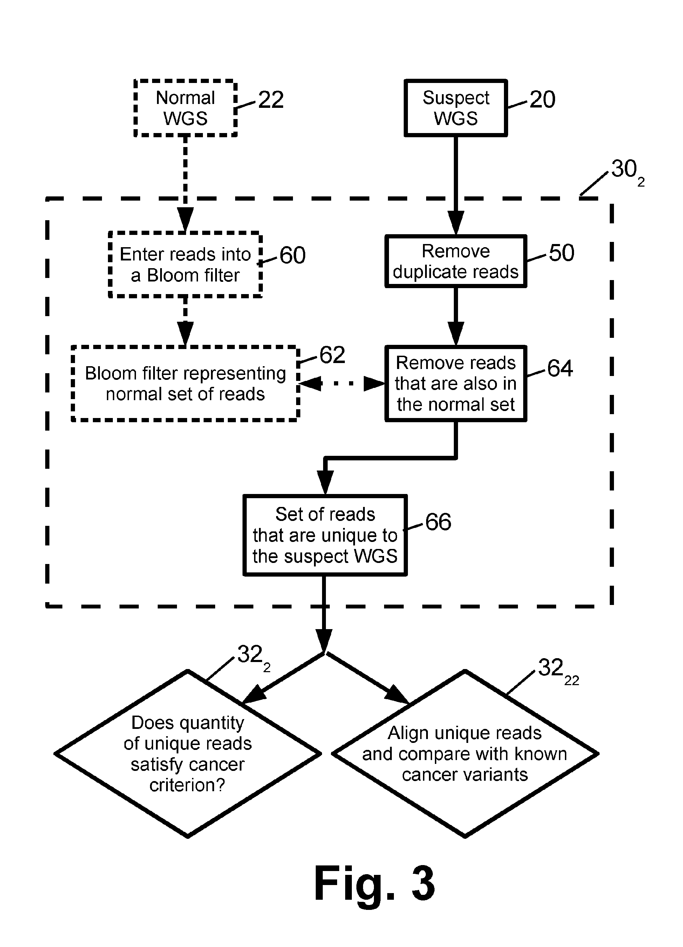

With reference to FIGURE 3, a second embodiment **30₂** of the WGS comparison metric computation operation **30** and a second embodiment **32₂** of the classifier operation **32** are described, which make more use of the available information. The operation **50** is performed as in the embodiment of FIGURE 2 in order to remove duplicate reads from the suspect WGS. On the normal WGS side, the reads are entered into a Bloom filter in an operation **60** to create a Bloom filter **62** representing the reads of the normal WGS **22**. As already noted, this has the effect of removing all duplicates from the normal WGS. In an operation **64**, each read of the suspect WGS is queried against the Bloom filter **62** in order to determine whether the read is part of the normal WGS **22**. The unique reads, that is, the reads that are unique to the suspect WGS **20** and are not included in the normal WGS **22**, are accumulated as a set of reads **66** that are unique to the suspect WGS.

In performing the operation **64**, the property that the Bloom filter never erroneously indicates that a read is not in the filter when it actually is ensures that the set of unique reads **66** does include not include any reads that are part of the normal WGS. However, it is possible that a few unique reads may be erroneously filtered out by the operation **64** since the Bloom filter **62** can erroneously indicate a read is in the filter when it is not. Thus, it is assured that the reads **66** are all unique to the suspect WGS **20**, although some unique reads may have been missed.

The set of unique reads **66** can be treated as the WGS comparison metric, or alternatively a WGS comparison metric can be derived from the set **66**. In the illustrative embodiment of FIGURE 3, a WGS comparison metric is derived from the set **66** as the quantity of unique reads which serves as input to the classifier **32₂** (preferably, the quantity of unique reads is normalized by the total number of reads in the suspect WGS **20** or by the total number of reads in the suspect WGS **20** after removal of duplicates via operation **50**). Another suitable WGS comparison metric is the ratio of total aligned length of the reads reads **66** that are unique to the suspect WGS **20** to the total genome length of the suspect WGS **20** (optionally after removal of duplicates as per operation **50**). This WGS comparison metric is an effective measure of the total change incurred in the cancer genome (assuming the suspect tissue is indeed cancer), and can be applied by the classifier **32₂** in place of unique reads quantity.

Alternatively, as also shown in FIGURE 3 as alternative decision operation **32₂₂**, the unique reads **66** can be aligned and compared with known cancer variants. In this approach, the unique reads (with duplicates removed) of the normal WGS **22** are collected in the Bloom filter **62**. If there are multiple normal tissue samples, they can be pooled in the Bloom filter **62** by inputting all the normal WGS reads from all the samples into the Bloom filter **62** as per operation **60**. The Bloom filter **62** thus represents a "Normal Set" of reads. This "Normal Set" is compared with a "Cancer Set" of reads obtained as the unique reads (as per operation **50**) of the suspect WGS **20**. Again, if multiple suspect tissue samples were sequenced, then the reads from these multiple samples can be pooled. (Here a Bloom filter is not suitable because there is no way to recall reads from a Bloom filter - it is only possible to query whether a given read is in the Bloom filter). The reads of the "Cancer Set" (that is, the output of operation **50** together with pooling of reads from multiple suspect tissue samples if provided) that also occur in the "Normal Set" are discarded (again, this is implemented in operation **64** by querying against the Bloom filter **62**). The remaining unique reads **66** are expected to be a "Causative Set" in that they contain the variants specifically associated with cancer. In the alternative classifier **32₂₂** these unique reads **66** are subjected to *de novo* alignment so as to identify single nucleotide polymorphisms (SNPs), Indels (insertions or deletions), or other genetic variants, and the identified variants are compared to cancer-correlative variants known in the literature. In this embodiment the use of the WGS comparison metric (which in this embodiment is the actual set of unique reads **66**) enables substantially faster processing because the bulk of the genome is not aligned and searched for probative variants. Instead, only those reads **66** that are not part of the standard reference sequence and are not variants of the normal genome of the specific subject **6** undergoing investigation are aligned and searched.

In the approach of FIGURE 3 alignment is performed only on the set of unique reads **66**. However, even if alignment of the suspect and normal WGS **20**, **22** is performed, substantial efficiency gains can be realized by employing a WGS comparison metric comprising or computed from the set of variants that are unique to the suspect WGS **20**.

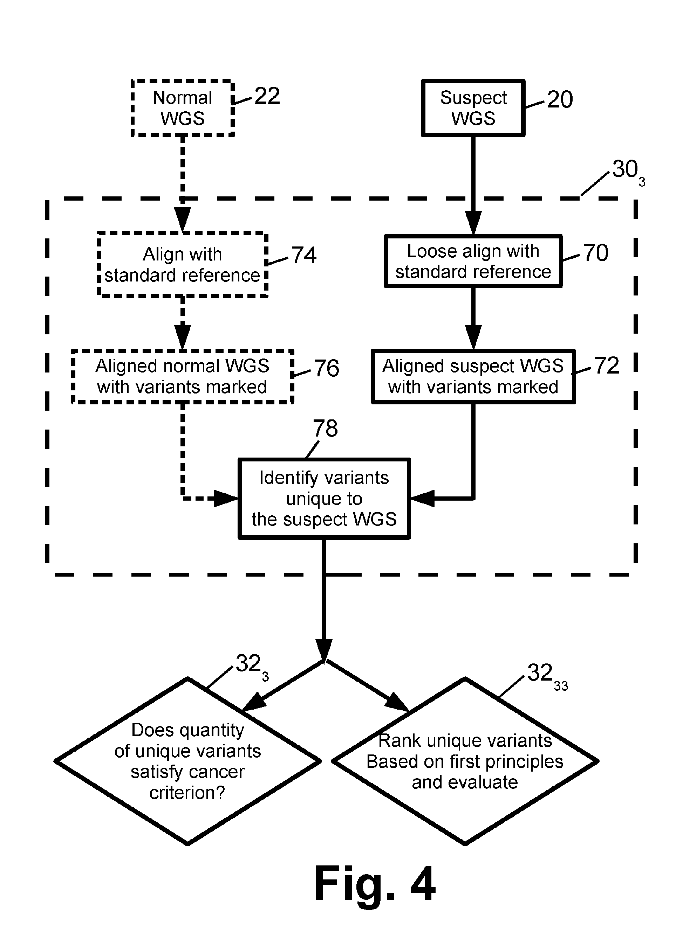

With reference to FIGURE 4, in an operation **70** the suspect WGS **20** is aligned with a standard reference sequence to produce an aligned suspect WGS **72** with variants (respective to the standard reference genome) marked. Similarly, in an operation **74** the normal WGS **22** is aligned with the standard reference sequence to produce an aligned normal WGS **76** with variants marked. The alignment **70** is preferably a "loose" alignment, that is, an alignment that is performed in a less stringent fashion so as not to reject the novel variants, which are expected to be present if the suspect tissue sample **10** is actually a cancer sample, as errors. In an operation **78**, the variants of the aligned suspect WGS **72** are filtered against the variants of the aligned normal WGS **76** to identify a set of variants that are unique to the suspect WGS **20**. The WGS comparison metric comprises or is computed based on this set of unique variants.

In one approach, the WGS comparison metric comprises the quantity of the unique variants found only in the suspect WGS (again, optionally normalized by the total number of variants in the aligned suspect WGS **72** or by another normalization factor). In the illustrative example, this WGS comparison metric serves as input to a classifier **32₃** which compares the quantity of the unique variants found only in the suspect WGS against a suitable cancer criterion. Typically, a higher number of unique variants in the suspect WGS **20** tends to suggest cancer, and so the cancer criterion employed by the classifier **32₃** is suitably a threshold above which the suspect tissue sample **20** is labeled as cancer.

In another approach also depicted as an alternative classifier **32₃₃** in FIGURE 4, the unique variants that are found only in the suspect WGS **20** are ranked according to impact level assessed based on the literature. For example, aberrations at or near oncogenes and tumor suppressor genes are assessed to have high impact, as are increasing telomere length. Tri and tetraalleleic single nucleotide variants (SNVs) are suitably tabulated to identify patterns suggesting local multiple tumor cell populations.

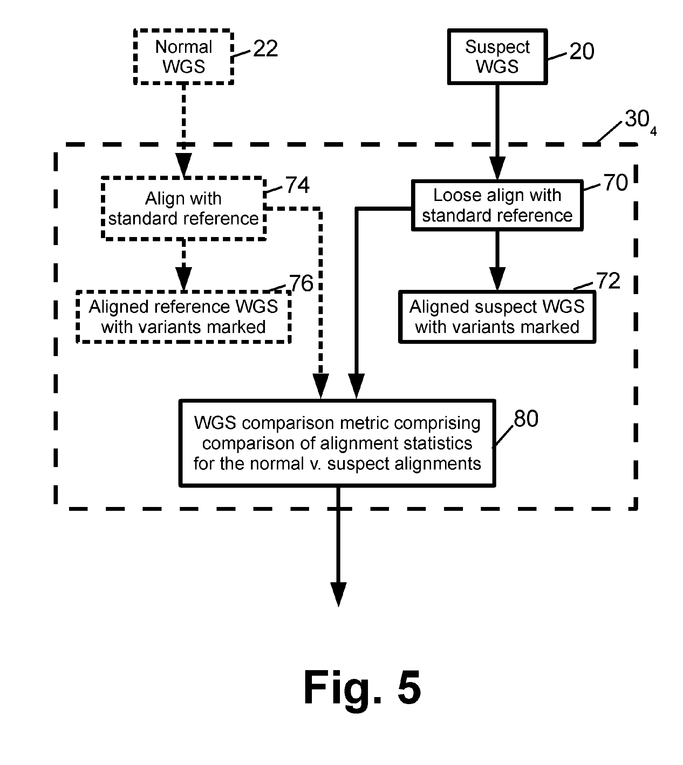

With reference to FIGURE 5, a fourth embodiment **30₄** of the WGS comparison metric computation operation **30** is described. This embodiment again employs the alignment operations **70**, **74** to generate the aligned suspect and normal WGS **72**, **76**. In this embodiment, alignment statistics generated by the alignment operations **70**, **74** are formulated into a WGS comparison metric in an operation **80**. Various alignment statistics are expected to effectively differentiate a cancer WGS versus a normal WGS. The inventors have observed that the four features of Table 1 are typically significantly different in cancer WGS as compared with normal WGS. Other parameters that are contemplated to be effective for discriminating these cell types include broken pair end, pair not found, pair orientation, and so forth.

With continuing reference to FIGURES 4 and 5 and with further reference back to FIGURE 1, it is noteworthy that the aligned suspect WGS **72** with variants (respective to the standard reference genome) marked corresponds to the output of the operation **40** shown in FIGURE 1. So, if the variant-based analysis **40**, **42**, **44**, **46** is to be performed conditional upon the test **30**, **32** outputting the result of cancer **36**, then operation **40** can be omitted and the aligned suspect WGS **72** can be directly input to operation **42**.

**Table 1: Read parameters observed in normal and cancer reads**

| Feature | Normal | Cancer |
|---|---|---|
| Unique (%) | 78.66 | 72.7 |
| No-specific matches (%) | 21.33 | 26.3 |
| Zero-coverage (%) | 24.3 | 11.4 |
| Coverage SD (Norm) | 1.18 | 2.6 |

The disclosed cancer tests based on WGS data provide fast assessment for pre-screening the massive WGS for probable genomic alterations attributable to cancer, thus providing a guide for computationally and time extensive analysis pipeline. The disclosed cancer tests are also expected to be useful for quantization of the progression of cancer. The disclosed cancer test embodiments effectively measure the genomic damage incurred due to the cancer on the scale of the entire WGS. These results are obtainable quickly without waiting for detailed specific variant-based genomic analysis. The disclosed cancer tests can be used to select defined analysis pipeline for cancer which is different from normal genome analysis, and employs a limited computational infrastructure. The WGS comparison metric is a suitable measure of the dedifferentiation/malignancy level of the cancer and thus is of prognostic value.

In some practical cancer diagnosis applications, suspect and normal tissue samples **10**, **12** are sequenced to the same coverage and the raw sequencing reads are used to measure the randomness of the cancer genome. The base-line (i.e., normal) WGS **22** for normal cells is prepared from the subject **6** by performing whole genome sequencing on normal tissue samples **12** which may, for example, be white blood cells (WBC), cells from the buccal cavity, or so forth. The suspect WGS **20** is obtained from cancerous cells sequencing. The raw reads are directly compared and the WGS difference metric obtained.

For detection of cancer progression, suspect tissue samples **10** are collected from different regions of the cancer tissue and boundary and also from involved lymph node or nodes in case of nodal progression of disease (where possible). Suspect tissue samples **10** may also be collected from metastatic foci (where possible and applicable). Normal tissue samples **12** are collected from appropriate normal tissue, such as normal lung tissue in the case of small cell lung carcinoma, or from a skin biopsy in case of basal cell carcinoma/cutaneous squamous cell carcinoma. The normal tissue samples **12** serve as a control or baseline.

Another application of the cancer cell identification approaches disclosed herein pertains to tumor delineation. As part of the planning process for surgical tumor removal, gamma knife surgery, or radiation therapy, the tumor should be accurately delineated. However, because cancer cells are closely related to, and hence may be difficult to distinguish from, normal body cells, such delineation can be difficult. Imaging techniques such as computed tomography (CT) or magnetic resonance imaging (MRI) may fail to provide a crisp delineation between the tumor and surrounding healthy tissue, and the imaged boundary (even if well defined in the image) may not precisely match the physical distribution of cancer cells due to microinfiltrations or the like. Histopathology can also be employed. Here, suspect tissue is extracted and examined microscopically, possibly in conjunction with probative staining, in order to differentiate and identify cancer cells. Histopathology is reliant upon the cancer cells having morphologically distinct characteristics and/or an identifiable coloration under appropriate staining conditions. Unfortunately, this is not always the case. Where the differentiation from normal cells is subtle, accurate histopathology assessment is reliant upon the skill of the human technician and hence is prone to human error. Indeed, in some cases the cancer cells may be morphologically identical with normal cells, making histopathology ineffective.

The rapid throughput provided by the disclosed cancer cell identification techniques facilitates the use of these techniques in tumor boundary delineation.

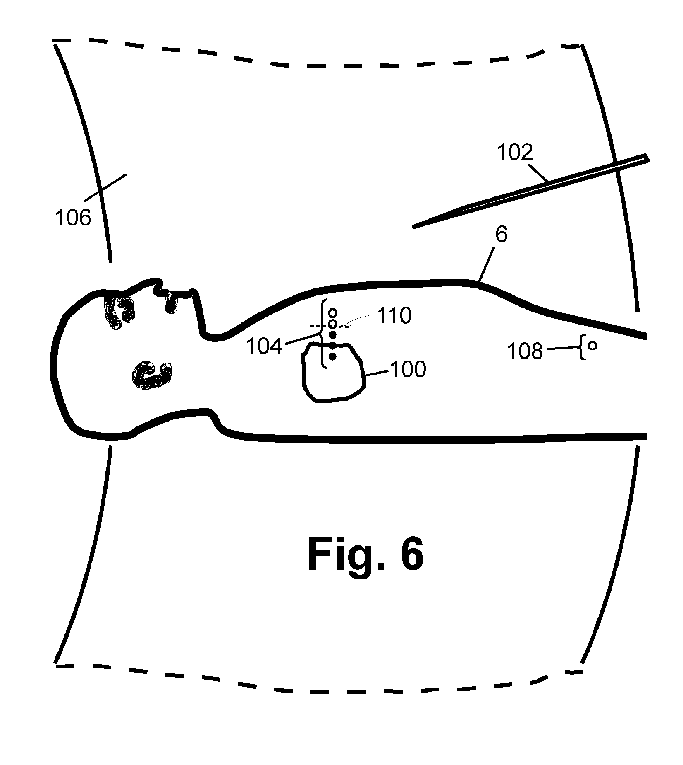

With reference to FIGURE 6, tissue samples are collected from the subject **6** at locations in and near a tumor **100** using image guided sample collection in which an interventional instrument **102** such as a biopsy needle or the like acquires tissue samples **104** under the guidance of an imaging system **106** (of which a portion of a scanner bore is diagrammatically shown). For sequencing of genomic DNA/mRNA the interventional instrument **102** is suitably an aspirated needle (which may be insufficient for certain types of histopathology). The sampling can employ any suitable acquisition technique, such as fine needle aspiration biopsy (for accessible tumors), stereotactic biopsy for neural tumors, or so forth. The imaging system **106** can be any modality capable of imaging salient features such as the tumor **100** and neighboring organs or other critical structures (not shown in FIGURE 6), such as computed tomography (CT) or magnetic resonance (MR). In some embodiments the imaging system **106** is the Brilliance^{™} Big Bore^{™} CT (available from Koninklijke Philips Electronics N.V., Eindhoven, The Netherlands) which has a large bore diameter that facilitates performing the interventional sample acquisition procedure. To employ the cancer cell identification techniques disclosed herein, at least one normal tissue sample **108** is also acquired from the subject **6**. In some embodiments the normal tissue sample **108** may be acquired by a mechanism other than the interventional instrument **102**, such as an oral swab in the case of an oral sample. For illustrative purposes, those samples **104** that comprise cancer tissue are shown as filled dots, while those samples **104**, **108** that comprise normal tissue are shown as open dots. (Of course, this is to be determined by the cancer cell test, except in the case of the reference normal sample **108**). Also shown in FIGURE 6 is an actual boundary **110** of the tumor **100**, where the boundary **110** separates normal tissue from cancer tissue. (Again, this boundary **110** is to be determined by the cancer cell tests on the acquired tissue sample **104**).

Once the tissue samples are collected, they are processed as disclosed herein with reference to FIGURES 1-5 (where each sample **104** corresponds to the suspect tissue sample **10** and the tissue samples **104** are processed independently, and the tissue sample or samples **108** is used as the normal tissue sample **12**) in order to classify each sample **104** as cancer tissue or normal tissue. Based on these classifications and the sample locations of from which the tissue samples **104** were acquired (these locations are recorded during tissue sample acquisition, for example using spatial coordinates provided by the imaging system **106**), the extent of the tumor **100** is spatially mapped and the boundary **110** between cancer tissue and normal tissue is determined. In generating the WGS, in some embodiments RNA genomic sequencing is generated (either instead of or in addition to DNA sequencing) using a suitable techniques such as exome capture.

In one approach, the tissue samples **104** are collected from different depths of the tumor radially outwards from center to outside the boundary indicated by imaging, as shown in FIGURE 6. To provide multidimensional (e.g., 2D or 3D) mapping, this is suitably repeated along one or more pairs of orthogonal diameters (such multi-dimensionality is not indicated in FIGURE 6). DNA and/or RNA from these samples is extracted and sequenced to generate a suspect WGS for each sample **104**.

In some embodiments, genetic variants such as single nucleotide polymorphisms (SNP's), indels, structural variants (SV's), copy number variants (CNV's), and so forth are extracted using conventional genetic analysis, expression patterns are extracted and compared against a database of signatures are reported to have association with the type of cancer corresponding to the tumor **100**. The resection boundary **110** is drawn across points where normal sequence patterns are observed.

However, it is generally not necessary to identify the type of cancer, as the nature of the tumor **100** is generally known before scheduling radiation therapy, gamma knife surgery, surgical tumor removal, or the like. Accordingly, the disclosed approach, e.g. as described herein with reference to operations **30**, **32** of FIGURE 1, is suitably employed and has the advantage of being substantially faster than conventional variant analysis.

In a variant approach, tissue samples **104** are collected as described with reference to FIGURE 6, and for each radially adjacent pair of samples along the radial line (working outwards from the center of the tumor **100**) the two WGS are compared with each other to identify the non-matching reads of the outer sample. These non-matching reads of the outer sample are selected and aligned against a reference sequence. The alignment is expected to be poor until the outward progression reaches a point where the outer sample of the pair is a sample of normal tissue - at that point the alignment should be good (e.g., quantified as the alignment percentage being above a stopping threshold).

In another variant approach, sample collection is as described with reference to FIGURE 6. However, instead of direct DNA sequencing, exome capture sequencing is performed to generate an RNA WGS. Transcriptome of normal samples is expected to be different from the cancer samples, thus enabling detection of the boundary **110**.

In another variant approach, sample collection is as shown in FIGURE 6 and employs image guidance using the imaging system **106**. In this variant approach, near real time sequencing of the transcriptome is performed by a sequencing methodology such as nanopore sequencing See http://www.nanoporetech.com/, last accessed October 27, 2011. The transcriptome analysis is optionally verified by reference to a database of expression signatures.

In another variant approach, image guided tissue sample collection is performed as described with reference to FIGURE 6 around the boundary of the tumor **100** as indicated by imaging within the range of a known (average) microinfiltration length for the tumor and beyond it in apparently normal tissue. Rapid WGS analysis is performed in accordance with one of the techniques described with reference to FIGURES 1-5 for all the samples **104** including the first normal sample identified outside the boundary **110**. More detailed or thorough sequencing (i.e., "deep sequencing") is then performed on the first normal sample identified outside the boundary **110** to verify that it is indeed normal tissue. If this deep sequencing indicates there is still some non-negligible contribution from malignant tissue, then this sample is included in the resectable area (i.e., the boundary **110** is expanded outward to encompass this sample). In the latter case, the process is optionally repeated with the next-outward sample that tested normal using the rapid WGS analysis, i.e. this next-outward sample is checked using deep sequencing.

In another variant approach, the sequencing reads from different tissue samples **104** are subtracted from each other. A percentage of variation within normal tissue is determined (e.g., using the normal tissue samples **108**). A variation of around 1.5-2.5% is generally expected for normal tissue. Cancer tissue samples are expected to exhibit a larger variation than normal tissue, thus enabling the boundary **110** to be detected. For example, in some such embodiments, if the reads similarity is less than 97.5% between two tissue samples, then it may be regarded as difference in cells types and the boundary **110** may be thusly defined.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A method comprising:
processing a suspect tissue sample **(10)** acquired from a subject **(6)** to generate a suspect whole genome sequence **(20)**;
processing a normal tissue sample **(12)** acquired from the subject to generate a normal whole genome sequence **(22)**;
computing **(30)** a whole genome sequence comparison metric comparing the suspect whole genome sequence with the normal whole genome sequence; and
identifying **(32)** whether the suspect tissue sample comprises cancer tissue based on the computed whole genome sequence comparison metric.

2. The method of claim 1, wherein the identifying **(32)** does not include identifying whether the tissue sample comprises any particular type of cancer tissue.

3. The method of claim **1**, wherein the identifying **(32)** does not include identifying any specific genetic variant in the suspect whole genome sequence.

4. The method of any one of claims **1-3**, wherein the identifying **(32)** comprises:
labeling the tissue sample as either cancer tissue or normal tissue based on the computed whole genome sequence comparison metric.

5. The method of any one of claims **1-4**, wherein the computing **(30₁)** comprises:
computing a metric of duplicate reads **(54)** in the suspect whole genome sequence **(20)**;
computing a metric of duplicate reads **(56)** in the normal whole genome sequence **(22)**; and
computing the whole genome sequence comparison metric based on the metric of duplicate reads in the suspect whole genome sequence and the metric of duplicate reads in the normal whole genome sequence.

6. The method of any one of claims **1-4**, wherein the computing **(30₂)** comprises:
determining a set of suspect genome-specific reads **(66)** that are (i) contained in the suspect whole genome sequence **(20)** and (ii) not contained in the normal whole genome sequence **(22)**;
wherein the whole genome sequence comparison metric comprises or is computed based on the set of suspect genome-specific reads **(66)**.

7. The method of any one of claims **1-4**, wherein the computing **(30₃)** comprises:
identifying a set of suspect genome variants by aligning **(70)** the suspect whole genome sequence **(20)** with a reference sequence;
identifying a set of normal genome variants by aligning **(74)** the normal whole genome sequence **(22)** with the reference sequence; and
identifying **(78)** a set of variants that are (i) contained in the set of suspect genome variants and (ii) not contained in the set of normal genome variants.

8. The method of any one of claims **1-4**, wherein the computing **(30₄)** comprises:
aligning **(70)** the suspect whole genome sequence **(20)** with a reference sequence;
aligning **(74)** the normal whole genome sequence **(22)** with the reference sequence;
and
computing the whole genome sequence comparison metric **(80)** based on comparison of alignment statistics for aligning the suspect whole genome sequence and alignment statistics for the aligning the whole genome sequence.

9. A non-transitory storage medium storing instructions executable by an electronic data processing device **(24)** to perform a method as set forth in any one of claims **1-8**.

10. An apparatus comprising:
an electronic data processing device **(24)** configured to perform a method as set forth in any one of claims **1-8**.

11. The method of any one of claims **1-8**, further comprising:
acquiring tissue samples **(104)** from the subject **(6)** at a plurality of sampling locations in or near a tumor **(100)**;
recording the sampling locations;
performing the processing, computing, and identifying for each tissue sample; and
delineating a boundary **(110)** of the tumor based on the identifying and the recorded sampling locations.

12. A method comprising:
classifying tissue samples **(104)** acquired from a subject **(6)** at sampling locations in or near a tumor **(100)** respective to cancer based on genetic testing of the tissue samples; and
delineating a boundary **(110)** of the tumor based on the classifying and knowledge of the sampling locations from which the samples were acquired.

13. The method of claim **12**, wherein the classifying comprises:
comparing genetic content of the tissue samples **(104)** with genetic content of normal tissue **(108)** of the subject, wherein the classifying does not include comparing genetic content of the tissue samples **(104)** with a genetic variant that has been shown in a clinical study to correlate with a type of cancer.

14. A method comprising:
acquiring a plurality of probative tissue samples **(104)** from a subject **(6)** in or near a tumor **(100)**;
recording the sampling locations of the probative tissue samples;
classifying each probative tissue sample respective to cancer based on genetic testing of the probative tissue sample; and
delineating a boundary **(110)** of the tumor based on the classifications of the probative tissue samples and the recorded sampling locations.

15. The method of claim **14**, further comprising:
acquiring a normal tissue sample **(108)** from the subject **(6)** at a location effective to ensure that the normal tissue sample does not comprise cancer tissue;
wherein the classifying of each probative tissue sample **(104)** comprises comparing genetic content of the probative tissue sample with genetic content of the normal tissue sample.
